# EUROPEAN PATENT SPECIFICATION

(11) **EP 0 699 414 B1**
(45) Date of publication and mention of the grant of the patent: **12.01.2005**
(21) Application number: 95305977.1
(22) Date of filing: 25.08.1995
(51) Int. Cl.: A61B 5/083

(54) **Disease management system**
Anordnung zum Behandlen von Krankheiten
Système destiné au traitement de maladies

(30) Priority: 30.08.1994 GB 9417421
(43) Date of publication of application: 06.03.1996
(73) Proprietor: The BOC Group plc, Windlesham Surrey GU20 6HJ (GB)
(72) Inventor: Walsh, Jeremy, Loughborough, Leicestershire LE11 0NY (GB); Williams, Deryk John Peter, Woking, Surrey GU21 1NW (GB)
(74) Representative: Bousfield, Roger James

(56) References cited:
- EP-A- 0 078 381
- EP-A- 0 647 427
- WO-A-93/05709
- WO-A-94/23650
- DE-A- 2 812 379

## Description

The invention relates to a system for the management of disease by measuring and monitoring the gaseous composition and rate of change of the composition of exhaled air of a patient. It relates in particular to a system for the management of lung disease.

The management of lung disease is increasing in importance due to the growing prevalence of certain diseases and of asthma in particular. This is believed to be due to decreasing air quality caused by higher pollution and toxic emissions worldwide. Diagnosis of asthma is historically more difficult compared with many other lung diseases because there are no objective diagnostic criteria for the disease. During an asthma attack, there is an inflammatory reaction leading to oedema and bronchospasm, reducing the ability of the lungs to absorb oxygen. As those characteristics are highly variable and transitory, it can be difficult to diagnose asthma or judge the severity of an attack.

DE-A-2 812 379 relates to a method and apparatus for pulmonary diagnosis, in particular for the diagnosis of pulmonary emphysema. The method involves simultaneous recording of tidal volume and of the concentration of a respiratory gas component during a breath, storage of inspiratory and expiratory tidal volume and expiratory concentration data, determination by integration of a section of the tidal volume curve, which corresponds to a defined concentration section as regards the initial and end-expiratory concentration difference, and recording of this tidal volume section as a function of the preceding inspiratory tidal volume over a range of inspiratory tidal volumes. Findings on pathological changes in the lung can be obtained in a simple manner from this data.

WO-A-93/05709 relates to a method and apparatus for ascertaining the function or condition of the lungs of a patient by measuring the nitric oxide content of the expiratory gas. The amount of nitrogen monoxide formed in the lungs during expiration and/or the time distribution of the amount of nitric oxide is compared with the maximal corresponding functions of a healthy patient and a deviation disclosed by the comparison can be interpreted as an impairment of the lung function.

Generally, a peak flow meter is used for indicating the severity of asthma attacks. This is used to measure the peak expiratory flow rate from the lungs of a patient which, during bronchospasm, is reduced. However, a peak flow meter has deficiencies in that it lacks precision; it requires the cooperation of the patient, which can be difficult in the case of young children, patients under sedation, in shock or in a coma; it requires the forced exhalation from the lungs which can increase the severity of bronchospasm; it cannot provide for the continuous monitoring of the asthma patient; and it is limited in its ability to be integrated into the management of the medication and hence the disease itself.

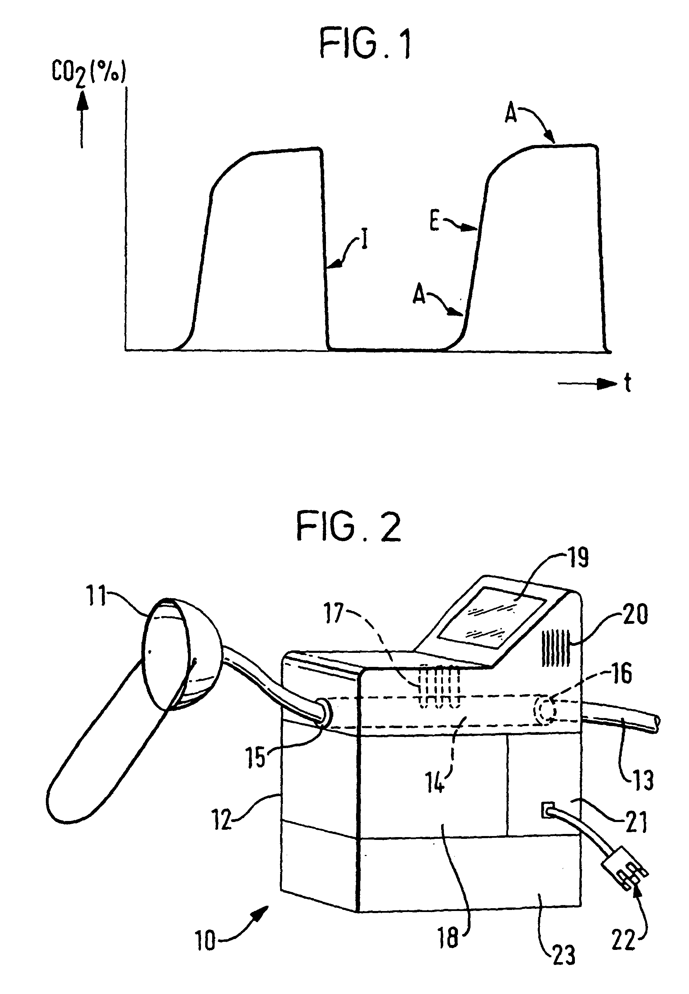

Some of these problems can be overcome by the use of capnography techniques in which a capnogram - a curve obtained by continuously recording the carbon dioxide partial pressure in a sample of exhaled breath - is produced. A normal capnogram has a substantially square wave pattern of the type shown in Figure 1 recording, (against time (t), the carbon dioxide level during inspiration (I) and expiration (E). The shape of the capnogram curve during the expiration phase, in particular the rate of change of the carbon dioxide level as evidenced by the slope of the capnogram (particularly in the vicinity between the arrows "A") can be a good indication of whether the patient's lungs are healthy or whether there is a manifestation of asthma and its severity.

WO-A-93/05709 discloses that the concentration and time-distribution of carbon dioxide in the air exhaled by a patent may be monitored. The shape of the curve of the carbon dioxide concentration versus time indicates the lung function or lung condition. A steeper curve indicates a better lung capacity or condition than a flatter curve. Capnography is a particularly relevant technique for indicating the degree of ventilation/perfusion mis-matching, a main problem associated with asthma.

Currently available medication and therapy is satisfactory to control some forms of lung disease when it is used correctly. However, certain conditions cannot be treated effectively. In the treatment of asthma the various pharmaceutical and therapy regimes can control all but the worst asthma attacks. However, often the asthma sufferer does not recognise the severity of the oncoming attack until suffering from marked or substantial bronchospasm. A contributing factor is the inadequacy of generally available testing and monitoring devices, because they do not accurately measure asthma.

According to the present invention there is provided a portable disease management system for monitoring the performance of a patient's lungs which comprises a first component adapted to measure velocity of expiratory flow exhaled from the lungs of a patient, a second component adapted to measure the concentration of carbon dioxide in the expiratory flow, a third component adapted to measure the rate of change of the concentration of carbon dioxide in the expiratory flow and electronic processing means for continuously recording the measurements over the exhalation periods of the patient's breathing cycle, for comparing signals received from the first, second and third components with reference data, [for example look-up tables], and for generating advisory signals indicative of the state of health of the patient.

In certain circumstances, it could be advantageous for the measurement of the rate of change of the carbon dioxide for one or more exhaled breaths by the patient to be normalised, for example with respect to the time axis with reference to a population norm or to an individual patient norm, for each respiratory cycle.

Whether or not the measurement of the rate of change of the composition of CO₂ is normalised, the measurement can be effected, for example:
i) by measuring the slope of a line tangential to a curve of the capnogram shown in Figure 1, particularly in the area between the arrows "A" but also in other parts of the curve, and/or
ii) by measuring the volume beneath that curve or part volumes beneath that curve, and/or
iii) taking other measurements and making correlations between the slope, the volume(s) or the other measurements, and/or
iv) by a Fourier transform to analyse the shape of the curve.

The system is however most suited to use as a lung disease management system in respect, for example, of asthma and chronic obstructive airway disease.

An important part of the invention is that the results (or signals) of the measurements, ie, the concentration and rate of change of CO₂ in the expiratory flow, are compared with reference data. By the term "reference data" is meant any figures, graphs or other means which can be used to compare the measurements to determine the health of the patient by use of the processing means.

For example, the reference data may simply be statistically produced figures for a normal, healthy person. Alternatively, they could be based on ones previously produced by the same patient to ascertain any change in that patient's health in particular.

The disease management system in accordance with the present invention includes electronic measurement and comparison means, in particular one which continuously records the flow measurements, analyses, compares and advises the user, through the generation of signals by the measurement devices and by the processing means, of either the gaseous make up over the normal breathing cycle of the exhalations of a disease sufferer or potential disease sufferer, and/or it advises on the appropriate therapy. As such, it is an "intelligent" multi-meter of, for example the lung, monitoring its performance through flow of, composition of, and rate of change of composition of inhalation and exhalation CO₂.

The system can also be programmed to take in to account previous data and the clinical outcome for an individual patient in order to provide enhanced performance of the system which is tailored to that patient's condition.

The signals generated by the processing means can be relayed to the user of the system in any convenient way, for example by way of a digital print-out or read-out, by way of a colour coded scheme, by audible means or whatever. Means to communicate the signals automatically to a central control could also be included. The processing means can also be adapted to co-operate with a medication unit such that the patient is given medication in response to the signals generated by it.

The comparison of the results of the measurements of CO₂ concentration/rate of change with the reference figures may also, if desired, take in to account other factors such as external factors unconnected with the patient, for example ozone or other toxic gas levels in the ambient atmosphere, the time of day or time of year.

This can be made more specific by having the patient inhale a gas mixture of known composition which does not contain normal (or abnormal) environmental contaminants and monitoring the composition changes in the expired gas. This will make comparisons across wide geographic areas more relevant.

If time factors are relevant in this respect, the system can usefully include a calendar/clock function.

In certain cases, it may be desirable to include in the system an initial test which provides a less sophisticated method of initially testing the patient's condition, for example by use of a peak flow meter in the case of asthma, and only proceeding to use the system of the invention in the event that the patient appears to fail the initial, less sophisticated method. Equally, the use of such a less sophisticated method in conjunction with the system of the invention may provide extra information or data in addition to that of the system of the invention alone.

For a better understanding of the invention, reference is now made, by way of exemplification only, to the accompanying Figure 2 which is a schematic representation of an embodiment of a lung disease management system in accordance with the invention.

Referring to Figure 2, there is shown a lung disease management system 10 for monitoring CO₂ exhaled from and inhaled into lungs of a patient and for comparing the results obtained from the monitoring with reference data.

The system 10 comprises a mask 11 adapted to locate over the mouth and nose of the patient, a housing 12 and an outlet tube 13. The housing 12 includes an upper passage 14 in communication with the mask 11 and the outlet tube 13 and is provided with seals 15,16 at opposite ends of the passage 14 so as to prevent egress of gas from the interface of the mask 11 and passage 14 and the interface of tube 13 and the passage 14. The passage 14 is provided with sensing elements 17 for generating first signals indicative of CO₂ concentration in the passage 14 and second signals indicative of the rate of change of CO₂.

The housing 12 also comprises a processing unit 18 for comparing the first, and second signals with normal signals indicative of reference data, ie composition of flow and rate of change of composition in a healthy person and for generating further signals indicative of the state of health of the lungs of the patient in comparison with reference data held in or supplied to the system.

In accordance with the invention, initial signals may be generated by one of the sensing elements 17 indicative of velocity of flow of the gas through the passage 14 and these signals are also compared with reference data to provide a further indication of the state of the lungs of the patient.

Also provided in the housing 12 are a display 19 and an audio unit 20 for providing visible and audible information corresponding to the further signals, interface circuitry 21 including a connector 22 for communicating the signals to a remote location, for example a hospital computer, and a battery 23. The signals communicated to the remote location may be in a real-time electronic format for recording on magnetic media for further computer analysis if required.

The tube 13 may be connected to a medication unit (not shown). In response to a signal indicative that the patient requires medication, which signal is transmitted to the medication unit from the processing unit 18 through the interface circuitry 21, the medication unit dispenses the required medication to the patient through the tube 13, the passage 14 and the mask 11.

In use of a system of the type shown in Figure 2, the system can usefully effect repeated measurements of the CO₂ concentration being sensed by the sensing elements 17 and the rate of change of CO₂.

As a generality, a rapid rate of change of the carbon dioxide composition indicates that the patent's lungs are healthy whereas a slow rate of change indicates that medication might be required, both in comparison with reference data held in or supplied to the system.

In conjunction with such preferred embodiments, the system can also if appropriate be used selectively depending on whether the velocity of flow is deemed high or low, in particular in comparison with reference data.

If the velocity of the flow is high - as a generality a good thing - the system can utilise supplementary equipment to measure the peak flow of expiratory flow of gas, for example by a peak flow meter, whose value is compared with normal values of peak flow and a signal is generated which is used to indicate whether the lungs of the patient under inspection are satisfactory or whether medication might be required. The signal may vary depending on the type of user interpreting the signal.

If the velocity of the flow is low, the system can utilise the equipment described above in relation to Figure 2 in which the composition of carbon dioxide and its rate of change and, again in comparison with reference data, generate a signal which indicates whether the patients lungs are healthy or not. Generally, a rapid rate of change indicates healthy lungs; a slow rate indicates that medication might be required and it is generally preferable in such cases that repeated measurements are taken of the composition of the exhaled gas component and its rate of change.

It will be appreciated that the invention can provide a portable lung disease management system which effects efficient monitoring of the state of health of lungs without the necessity for forced exhalation of gas or cooperation of a patient.

The system may be provided with a control panel for controlling output from the processing unit 18 so that information provided by the system is selectively controlled. In this manner, the information may be controlled so that only some of the information is available for one category of user but other, or more, of the information is available for another category of user.

The system may be arranged to record lung management data such that it may be used in a conditional probability equation to record and advise as to the likelihood of developing an asthma attack.

The system may be arranged to record and monitor inhaled gases to advise the patient as to deterioration of air quality likely to increase the probability of an asthma attack.

The system may be used in conjunction with other monitoring means for the lungs or for other parts/organs of the body, for example a spirometer or pulse oximeter to provide an enhanced diagnostic capability for the patient as a whole.

## Claims

1. A portable disease management system for monitoring the performance of a patient's lungs which comprises a first component adapted to measure velocity of expiratory flow exhaled from the lungs of a patient, a second component adapted to measure the concentration of carbon dioxide in the expiratory flow, a third component adapted to measure the rate of change of the concentration of carbon dioxide in the expiratory flow and electronic processing means for continuously recording the measurements over the exhalation periods of the patient's breathing cycle, for comparing signals received from the first, second and third components with reference data, and for generating advisory signals indicative of the state of health of the patient.

2. A portable disease management system according to Claim 1, in which the measurement of the rate of change of the concentration of the carbon dioxide for one or more exhaled breaths by the patient is normalised with respect to the time axis with reference to a population norm or to an individual patient norm, for each respiratory cycle.

3. A portable disease management system according to Claim 1 or Claim 2, which additionally advises on the appropriate therapy.

4. A portable disease management system according to any preceding Claim, in which the processing means is adapted to co-operate with a medication unit such that the patient is given medication in response to the signals generated by it.

## Patentansprüche

1. Tragbares Krankheitsbeherrschungssystem zur Überwachung der Leistung der Lungen eines Patienten, das eine erste Komponente zum Messen der Geschwindigkeit der aus den Lungen eines Patienten ausgeatmeten Ausatmungsströmung, eine zweite Komponente zum Messen der Konzentration von Kohlendioxid in der Ausatemströmung, eine dritte Komponente zu messen der Änderungsrate der Kohlendioxidkonzentration in der Ausatemströmung, und elektronische Verarbeitungsmittel zum kontinuierlichen Aufzeichnen der Messungen über die Ausatemperioden des Atemzyklus des Patienten, zum Vergleich von von der ersten, der zweiten und der dritten Komponente empfangenen Signale mit Referenzdaten, und zum Erzeugen von dem Gesundheitszustand des Patienten anzeigenden Hinweissignalen umfasst.

2. Tragbares Krankheitsbeherrschungssystem nach Anspruch 1, wobei die Messung der Änderungsrate der Kohlendioxidkonzentration für eine oder mehrere Ausatmungen des Patienten bezüglich der Zeitachse mit Bezug auf eine Bevölkerungsnorm oder eine individuelle Patientennorm für jeden Atemzykus normalisiert wird.

3. Tragbares Krankheitsbeherrschungssystem nach Anspruch 1 oder Anspruch 2, das zusätzlich eine Empfehlung über die geeignete Therapie gibt.

4. Tragbares Krankheitsbeherrschungssystem nach einem der vorhergehenden Ansprüche, wobei die Verarbeitungsmittel dafür ausgelegt sind, mit einer Behandlungseinheit zusammenzuwirken, derart, daß dem Patienten in Abhängigkeit von den durch sie erzeugten Signalen eine Behandlung erteilt wird.

## Revendications

1. Système de mesures diagnostiques et d'attitude thérapeutique portatif destiné au monitorage des performances des poumons d'un patient, qui comprend un premier composant apte à mesurer la vitesse du flux expiratoire exhalé des poumons d'un patient, un deuxième composant apte à mesurer la concentration de dioxyde de carbone dans le flux expiratoire, un troisième composant apte à mesurer le rythme de changement de la concentration de dioxyde de carbone dans le flux expiratoire, et des moyens électroniques de traitement pour enregistrer en continu les mesures pendant les périodes d'expiration du cycle respiratoire du patient, pour comparer des signaux reçus des premier, deuxième et troisième composants avec des données de référence, et pour émettre des signaux de renseignements indicatifs de l'état de santé du patient.

2. Système de mesures diagnostiques et d'attitude thérapeutique portatif selon la revendication 1, dans lequel la mesure du rythme de changement de la concentration du dioxyde de carbone pour une ou plusieurs expirations du patient est normalisée par rapport à l'axe des temps en référence à une norme pour une population ou à une norme pour un patient individuel, pour chaque cycle respiratoire.

3. Système de mesures diagnostiques et d'attitude thérapeutique portatif selon la Revendication 1 ou la Revendication 2, qui fournit en plus des recommandations sur la thérapie appropriée.

4. Système de mesures diagnostiques et d'attitude thérapeutique portatif selon l'une quelconque des Revendications précédentes, dans lequel les moyens de traitement sont aptes à coopérer avec une unité de médication de telle sorte que le patient reçoive une médication en réponse aux signaux qu'ils émettent.
